(19) Europäisches Patentamt
European Patent Office
Office européen des brevets

(11) EP 0 661 056 B1

(12) **FASCICULE DE BREVET EUROPEEN**

(45) Date de publication et mention de la délivrance du brevet:
**28.05.2003 Bulletin 2003/22**

(51) Int Cl.⁷: **A61K 35/30**, A23L 1/30, A23L 1/312

(21) Numéro de dépôt: **94402818.2**

(22) Date de dépôt: **08.12.1994**

# (54) Suppléments alimentaires pour la nutrition des très jeunes enfants, contenant des phospholipides extraits de cerveaux

Ergänzungsnahrung für Säuglinge enthaltend Gehirnphospholipden

Feed supplement for the nutrition of newly-born children containing phospholipids extracted from the brain

(84) Etats contractants désignés:
**AT BE CH DE DK ES FR GB GR IE IT LI LU NL PT SE**

(30) Priorité: **31.12.1993 FR 9315965**

(43) Date de publication de la demande:
**05.07.1995 Bulletin 1995/27**

(73) Titulaire: **INSTITUT DE RECHERCHE BIOLOGIQUE S.A.**
**F-78870 Bailly (FR)**

(72) Inventeur: **Ponroy, Yves**
**F-78000 Versailles (FR)**

(74) Mandataire: **Burtin, Jean-François**
**Cabinet GEFIB,**
**55, rue Aristide Briand**
**92300 Levallois-Perret Cedex (FR)**

(56) Documents cités:
**WO-A-92/22291** **FR-A- 1 326 326**
**FR-M- 1 957**

- **PATENT ABSTRACTS OF JAPAN vol. 11, no. 206 (C-433) 3 Juillet 1987 & JP-A-62 029 951 (SHOWA YAKUHIN KAKO KK) 7 Février 1987**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 661 056 B1

## Description

La présente invention se rapporte au domaine de l'alimentation et notamment au domaine de la supplémentation de la nourriture en éléments nécessaires ou utiles.

Elle a plus particulièrement pour objet, de nouvelles compositions alimentaires destinées à compenser des déficits en acides gras essentiels dans l'alimentation de sujets fragiles ou malnutris.

On sait en particulier que les besoins spécifiques de la femme enceinte en acide gras essentiels et en particulier en acide α-linolénique (18:3 n-3) sont souvent mal assurés car l'alimentation et principalement les matières grasses usuelles sont pauvres en de tels acides et l'apport quotidien est faible pour ne pas dire insuffisant. Il peut en résulter des implications néfastes dans la maturation cérébrale du foetus, ou du nouveau né, plus particulièrement chez les enfants prématurés. Des articles récents, parus dans la littérature (cf. Crawford Ann. J. Clin. Nutr. (1993) 57 (suppl) - p.703-710), ont souligné l'importance du rôle joué par ces acides gras polyinsaturés d'un type particulier, pour le développement des cellules nerveuses et la constitution des parois de leurs membranes.

Il s'y ajoute le fait que les enfants prématurés et les enfants de faible poids à la naissance, ont des besoins plus importants en acides gras polyinsaturés que les autres nourrissons, qui ne sont satisfaits ni par les laits artificiels actuels, qui en contiennent peu, ni par le lait maternel étant donné que les mères allaitantes sont souvent carencées en de tels acides gras.

En outre, lorsque les laits artificiels contiennent une quantité suffisante d'acide linoléique ($C_{18}$:2 n-6) et d'acide α-linolénique ($C_{18}$:3 n-3) l'équipement enzymatique des prématurés en enzymes d'utilisation (Δ6-desaturases) est trop faible pour les convertir en homologues supérieurs et, en particulier, en acide docosahexenoique ($C_{22}$:6 n-3), et en acide arachidonique ($C_{20}$-4 n-6). Un article récent de M. FIREMAN (Ann. J. Clin. Nutr. (1993) 57(suppl.) - p.829) fait le point sur ce problème d'équipement enzymatique.

Le problème de la présente invention est donc de réaliser un apport d'une quantité nécessaire et suffisante en ces deux acides gras qui apparaît primordial pour assurer un développement correct de la vision (P.PEIRANO J. Pédiatr. 120 (1992) 168-180) chez les prématurés ainsi que pour assurer une régulation satisfaisante de la croissance des autres fonctions cérébrales chez les prématurés qui ne peuvent être nourris au sein.

Les essais effectués par les scientifiques américains (D.R HOFFMANN Ann. J. Clin. Nutr. (1993) 57(suppl.) p.807-812), ont montré que les fonctions de réception de la lumière chez les enfants prématurés subissent une maturation optimale lorsqu'un apport suffisant et assimilable d'acides gras, à longue chaîne polyinsaturée, appartenant à la série n-3, était réalisé, soit par ajout de lait maternel, soit par incorporation à l'alimentation d'huiles de chair de poissons.

On a même pu mettre en évidence (K.BJERVE Ann. J. Clin. Nutr. (1993) 57(suppl.) p.801-806S) une corrélation entre le taux d'acide docosahexenoique (DHA) dans le plasma des nouveau-nés et leur degré de prématurité, évalués selon des index de développement psychomoteur et mental.

Le problème consistait donc à trouver des sources d'acide docosahexenoïque appropriées. On a commencé par l'huile de chair de poisson mais celle-ci a l'inconvénient de contenir un fort pourcentage d'acide eicosapenténoïque (EPA $C_{20}$-5 n-3) dont la présence peut être dangereuse chez l'enfant et que l'on ne trouve pas normalement dans le lait maternel (cf. K.BJERVE ).

Dans le brevet WO 92 22291, un aliment pour bébé est décrit, contenant des extraits de lipides cérébraux. Cependant, le procédé d'extraction utilisé est tel que les phospholipides sont éliminés lors de l'extraction. Les extraits de cerveaux de mammifères de WO 92 22291 ne contiennent donc pas (ou très peu) de phospholipides.

L'invention réside en conséquence dans le fait que l'on peut apporter une source physiologique en acides gras polyinsaturés de la série n-3 et en particulier du DHA, en proportion physiologique, susceptible d'être utilisée telle quelle ou d'entrer dans la composition d'un lait infantile ou d'un supplément nutritionnel pour enfants prématurés ou nourrissons de faible poids à la naissance.

L'invention a donc pour objet des compositions alimentaires directement incorporables dans la nourriture des très jeunes enfants caractérisées en ce qu'elles renferment, en tant qu'apport alimentaire, des phospholipides extraits de cerveau de mammifères, à l'exception du cerveau humain, en association ou en mélange avec un support alimentaire approprié pour la nourriture de très jeunes enfants.

En particulier, les phospholipides de cerveau de mammifère sont les phospholipides cérébraux de porc. Ils ont la structure chimique suivante :

$CH_2O$ —— acide gras polyinsaturé à longue chaîne
$CH_2O$ —— acide gras polyinsaturé à longue chaîne
$CH_2O$ —— acide phosphorique - base aminée

La base aminée est, selon les phospholipides : la choline, la sérine, l'éthanolamine ou l'inositol.
Le cerveau des mammifères et tout particulièrement celui du porc contient une proportion assez constante en phospholipides suivants :

| | |
|---|---|
| sphingomyeline | 4 -7% |
| phosphatidylcholine | 20- 30% |
| phosphatidylsérine | 17 - 25% |
| phosphatidylinositol | |
| phosphatidyléthanolamine | 30 - 40% |

Ces phospholipides cérébraux ont une spécificité qui les distingue nettement des autres sources de phospholipides, animales ou végétales comme par exemple, les lecithines animales ou de soja. Cette spécificité tient, en particulier, à la nature chimique des acides gras qui les composent.
L'analyse des acides gras qui estérifient la molécule de glycérol montre qu'ils se répartissent comme suit, en moyenne :

| | | | |
|---|---|---|---|
| Acide oléique | 18:1 n-9 | environ | 25% |
| Acide linoléique | 18:2 n-6 | inférieur à | 1% |
| Acide γ-linolénique | 18:3 n-6 | inférieur à | 0,5% |
| Acide arachidonique | 20:4 n-6 | environ | 8,5% |
| Acide en | 22:4 n-6 | environ | 4% |
| Acide en | 22:5 n-6 | environ | 0,8% |
| Acide α-linolénique | 18:3 n-3 | | |
| Acide stéaridonique | 18:4 n-3 | | |
| EPA (acide eicosapentaénoïque) | 20:5 n-3 | inférieur à | 1% |
| Acide en | 22:5 n-3 | inférieur à | 1% |
| DHA (acide docosahexénoïque) | 22:6 n-3 | environ | 9% |

Les expériences effectuées ont indiqué que les acides gras contenus dans les phospholipides cérébraux de mammifères présentent l'avantage, en vue de la supplémentation des prématurés :

- de contenir environ 10% de DHA (22:6 n-3)
  et seulement 0,3% d'EPA (20:5 n-3)

- du fait que le rapport des acides gras polyinsaturés à longues chaînes, par rapport à ceux à chaîne plus courte (AGPI-LC) est satisfaisant :

$$\frac{\text{AGPI-LC n-6}}{\text{AGPI-LC n-3}} = 1,5$$

  En FRANCE, ce rapport est d'environ 2 dans le lait de femme (cf. G.DURAND Europ. J. Clin. Nutr. (1993) 47 (suppl.) p.700-710).

- de ce que les phospholipides cérébraux contiennent 8 à 9% d'acide arachidonique (20:4 n-3) qui, comme le DHA, apparaît essentiel pour le cerveau du prématuré.

- de ce que les phospholipides cérébraux contiennent près de 25% d'acides gras polyinsaturés supérieurs à C18.

- Les phospholipides cérébraux selon l'invention sont donc susceptibles d'apporter à l'enfant prématuré des doses physiologiques et équilibrées des différents acides gras dont il a besoin pour sa maturation cérébrale.
  Des études récentes apportent la preuve qu'un apport de phospholipides cérébraux, selon l'invention, provoque une augmentation du DHA (22:6 n-3) au niveau des structures cérébrales et de la rétine de façon plus efficace qu'un apport de triglycérides riches en acide α-linolénique (18:3 n-3).

L'invention concerne aussi un procédé d'obtention des phospholipides cérébraux et notamment de ceux de

porc, qui consiste à prélever par des moyens mécaniques sur des animaux fraîchement abattus, les cervelles, de les congeler immédiatement à -20°C et de les conserver à cette température, de laisser remonter ensuite la température des organes à une température comprise entre -5 et 0°C avant de les hacher dans un hachoir industriel puis dans des broyeurs, de façon à obtenir une pâte fluide dont la teneur en eau est d'environ 80%, d'éliminer l'eau des tissus en transférant la pâte fluide ainsi obtenue au sommet d'une chambre d'atomisation et d'évaporer, rapidement, l'eau dans un courant d'air surchauffé à 190-195°C, de séparer de la poudre résultante, la fraction lipidique en l'introduisant dans un mélange d'hydrocarbures aliphatiques en $C_6$ et en la maintenant sous agitation à température ordinaire, puis à filtrer le mélange obtenu et en concentrant la phase liquide séparée, sous pression réduite, pour obtenir une pâte brute que l'on coule dans de l'acétone préalablement additionnée d'un agent antioxydant alimentaire, que l'on sépare le précipité formé par filtration sous atmosphère d'azote et à sécher sous vide le produit pulvérulent ainsi recueilli.

La poudre de phospholipides peut ensuite être diluée ou incorporée dans des supports ou véhicules inertes aptes à l'alimentation des très jeunes enfants. On pourra ainsi diluer les phospholipides cérébraux avec un support digestible comme du lactose, de la caséine, de la poudre de lait ou des farines prédigérées. Selon le cas, le support est susceptible d'apporter des calories en faibles quantités ou en quantités plus importantes.

Les phospholipides selon l'invention peuvent également être formulés sous forme liquide et notamment, en suspension dans un véhicule aqueux comme par exemple, de l'eau de table, de l'eau sucrée ou des véhicules équivalents.

Les phospholipides cérébraux selon l'invention peuvent également être formulés sous forme d'émulsions et notamment de liposomes. Les liposomes peuvent être constitués de particules dont la taille s'échelonne ou est en majorité dans une zone de 100 à 500 μm. Ils peuvent rester en phase liquide ou bien être déshydratés pour être mélangés à un diluant alimentaire solide.

Les suppléments alimentaires ainsi réalisés peuvent, en outre, être additionnés d'autres facteurs de croissance tels que des vitamines (vitamines A, D ou vitamines du groupe B, des éléments minéraux, des facteurs biologiques, des acides aminés essentiels.

On pourra citer comme éléments minéraux, notamment des sels de calcium, de magnésium, de zinc ou de fer qui jouent un rôle dans le bien-être de très jeunes enfants.

Comme facteurs biologiques de croissance on pourra citer, en particulier, la taurine, la choline, l'inositol hexaphosphate de calcium.

Comme acide aminé essentiel, on pourra citer l'arginine ou la lysine.

Les suppléments alimentaires selon l'invention peuvent contenir de 1 à 20% de phospholipides cérébraux et sont utilisés par dilution dans les biberons si le mélange est totalement soluble ou sous forme de gouttes de suspension aqueuse ou d'émulsion. Cette dernière forme d'utilisation est préférée et permet une détermination plus précise de la quantité de phospholipides cérébraux selon l'invention, ajoutée en supplément à la nourriture des enfants en très bas âge.

Les exemples suivants illustrent l'invention. Ils ne la limitent en aucune façon.

## EXEMPLE I

| | |
|---|---|
| Phospholipides cérébraux de porcs en poudre | 25 g |
| Caséïne de lait | 20 g |
| Extrait de levure de bière | 0,5 g |
| Amidon de blé | 12 g |
| α-tocophérol | 0,2 g |
| Carbonate de calcium | 2,3 g |

pour 100 sachets de poudre contenant 0,25 g de phospholipides à incorporer au biberon ou à diluer dans un peu d'eau sucrée.

## EXEMPLE II

| | |
|---|---|
| Phospholipides cérébraux de porcs en poudre | 40 g |
| Hydroxy éthyl cellulose | 2 g |

(suite)

| | |
|---|---|
| Copolymère d'oxyde d'éthylène/oxyde de propylène commercialisé sous la marque Pluronic F18 | 8 g |
| Vitamine B12 | 5 mg |
| Gluconate de calcium | 0,500 g |
| Gluconate ferreux | 0,250 g |
| Palmitate d'ascorbyle | 0,100 g |
| Alginate de sodium | 4 g |
| Eau qsp | 100 ml |

l'émulsion ainsi réalisée est destinée à être incorporée au lait du nouveau-né.

**EXEMPLE III**

| | |
|---|---|
| Phospholipides cérébraux de porcs en poudre | 25 g |
| Lait en poudre écrémé | 25 g |
| Phosphate tricalcique | 2 g |
| Carbonate de fer | 1 g |
| Carbonate de manganèse | 1 g |
| Complexe vitaminique B | 0,2 g |
| Cellulose microcristalline | 26 g |

La poudre ainsi obtenue peut être répartie en sachets de 0,100 g dans le lait des nouveau-nés.

**EXEMPLE IV**

| | |
|---|---|
| Phospholipides cérébraux de porcs en poudre | 45 g |
| Hydroxy éthyl cellulose | 2 g |
| Copolymère d'oxyde d'éthylène/oxyde de propylène commercialisé sous la marque Pluronic F18 | 8 g |
| Vitamine E | 0,3 g |
| Alginate de sodium | 4 g |
| Eau qsp | 100 ml |

L'émulsion ainsi réalisée est destinée à être incorporée au lait du nouveau-né.

## Revendications

1. Compositions alimentaires directement incorporables dans la nourriture de très jeunes enfants **caractérisées en ce qu'**elles renferment en tant que constituant principal, des phospholipides extraits de cerveaux de mammifères à l'exception de ceux de l'homme, en association ou en mélange, avec un support alimentaire approprié pour l'alimentation de très jeunes enfants.

2. Compositions alimentaires selon la revendication 1° **caractérisées en ce que** le support alimentaire est un matériau digestible faiblement calorifique ou plus fortement calorique.

3. Compositions alimentaires selon la revendication 1° ou la revendication 2° dans lesquelles le support alimentaire est choisi parmi les éléments nutritifs choisi dans le groupe du lactose, de la caséïne, du lait en poudre, et des farines prédigérées.

**4.** Compositions alimentaires selon la revendication 1° dans lesquelles le support alimentaire est un véhicule aqueux sucré ou non sucré.

**5.** Compositions alimentaires selon la revendication 1° dans lesquelles les phospholipides cérébraux sont formulés sous forme d'émulsions.

**6.** Compositions alimentaires selon la revendication 1° dans lesquelles les phospholipides cérébraux sont formulés sous forme de liposomes dont la taille s'échelonne de 100 à 500 µm.

**7.** Compositions alimentaires selon l'une des revendications 1 à 6° **caractérisées en ce qu'**elles sont additionnées d'autres facteurs de croissance.

**8.** Compositions alimentaires selon la revendication 7° dans lesquelles le facteur de croissance est une vitamine ou un mélange de vitamines.

**9.** Compositions alimentaires selon la revendication 7° dans lesquelles le facteur de croissance est un élément minéral choisi dans le groupe formé des sels de calcium, des sels de magnésium, des sels de zinc et des sels de fer.

**10.** Compositions alimentaires selon l'une des revendications 1 9° dans lesquelles la teneur en phospholipides cérébraux s'échelonne de 1 à 20%.

**11.** Procédé d'obtention des phospholipides cérébraux selon la revendication 1° qui consiste à prélever, par des moyens mécaniques, sur des animaux fraîchement abattus, les cervelles, à les congeler immédiatement à -20°C et à les conserver à cette température, à laisser remonter ensuite la température des organes à une température comprise entre -5° et 0°C, avant de les hacher dans un hachoir industriel puis dans des broyeurs, de façon à former une pâte fluide dont la teneur en eau est d'environ 80%, d'éliminer l'eau des tissus en transférant la pâte fluide au sommet d'une chambre d'atomisation et en évaporant rapidement l'eau dans un courant d'air surchauffé à 190-195°C, à séparer de la poudre résultante la fraction lipidique par épuisement par un mélange d'hydrocarbures aliphatiques en $C_6$ par agitation, à température ordinaire, à filtrer le mélange obtenu et à concentrer à sec la phase liquide séparée sous pression réduite pour obtenir une pâte brute que l'on coule dans de l'acétone, à séparer le précipité formé par filtration sous atmosphère d'azote et à sécher, sous vide, le produit pulvérulent ainsi recueilli.

**12.** Utilisation des phospholipides cérébraux obtenus selon le procédé de la revendication 11° en vue de la réalisation de compositions alimentaires destinées à compenser les déficits en acides gras essentiels de l'alimentation des enfants prématurés ou malnutris.

**Patentansprüche**

**1.** In die Säuglingsnahrung direkt einbaubare Nahrungssubstanzen, **dadurch gekennzeichnet, dass** sie als Hauptbestandteil Phospholipoide, die aus dem Gehirn von Säugetieren mit Ausnahme von menschlichem Gehirn extrahiert wurden, als Beigabe oder Gemisch mit einem für die Säuglingsernährung geeigneten Nahrungsmittelträger enthalten.

**2.** Nahrungssubstanzen nach Anspruch 1, **dadurch gekennzeichnet, dass** der Nahrungsträger ein verdauliches, leicht oder stärker kalorienreiches Material ist.

**3.** Nahrungssubstanzen nach Anspruch 1 oder Anspruch 2, bei denen der Nahrungsträger aus den Nahrungselementen der Gruppe gewählt wird, in der Laktose, Kasein, Trockenmilch und vorverdaute Mehle enthalten sind.

**4.** Nahrungssubstanzen nach Anspruch 1, in denen der Nahrungsträger ein gezuckerter oder ungezuckerter Träger ist.

**5.** Nahrungssubstanzen nach Anspruch 1, in denen die Gehirnphospholipoide in Form von Emulsionen formuliert sind.

**6.** Nahrungssubstanzen nach Anspruch 1, in denen die Gehirnphospholipoide in Form von Liposomen formuliert

sind, deren Durchmesser 100 bis 500 µm misst.

7. Nahrungssubstanzen nach einem der Ansprüche 1 bis 6, **dadurch gekennzeichnet, dass** ihnen andere Wachstumsfaktoren beigegeben werden.

8. Nahrungssubstanzen nach Anspruch 7, bei denen der Wachstumsfaktor ein Vitamin oder ein Vitamingemisch ist.

9. Nahrungssubstanzen nach Anspruch 7, bei denen der Wachstumsfaktor ein mineralisches Element ist, das aus der Gruppe gewählt wird, die aus Kalziumsalzen, Magnesiumsalzen, Zinksalzen und Eisensalzen gebildet wird.

10. Nahrungssubstanzen nach einem der Ansprüche 1 bis 9, in denen der Gehalt an Gehirnphospholipoiden 1 bis 20 % beträgt.

11. Verfahren zur Gewinnung von Gehirnphospholipoiden nach Anspruch 1, welches darin besteht, dass man mit mechanischen Mitteln an frisch geschlachteten Tieren die Gehimmasse entnimmt, sie unverzüglich auf minus 20 °C einfriert und sie bei dieser Temperatur konserviert, dann die Temperatur der Organe auf eine Temperatur zwischen minus 5 °C und 0 °C ansteigen lässt, bevor man sie in einem industriellen Grobzerkleinerer und dann in einem Feinzerkleinerer dergestalt zerkleinert, dass eine flüssige Masse entsteht, deren Gehalt an Wasser ungefähr 80 % beträgt, wobei das Wasser aus den Geweben dadurch entfernt wird, dass man die flüssige Masse auf die Oberseite einer Zerstäubungskammer bringt und das Wasser in einem auf 190-195 °C erhitzten Luftstrom schnell verdampfen lässt, von dem entstehenden Pulver die Lipoidfraktion durch Auslaugen mit einem Gemisch aus aliphatischen $C_6$-Kohlenwasserstoffen unter Rühren bei Zimmertemperatur abtrennt, das erhaltene Gemisch filtert und die unter vermindertem Druck abgetrennte flüssige Phase zur Trockne eindickt, um eine Rohpaste zu erhalten, die man in Azeton gibt, wobei der gebildete Niederschlag durch Filtration unter Stickstoffatmosphäre abgetrennt und das so erhaltene pulverige Erzeugnis im Vakuum getrocknet wird.

12. Benutzung von Gehimphospholipoiden, die nach dem Verfahren gemäß Anspruch 11 erhalten wurden, zur Herstellung von Nahrungssubstanzen, die dazu bestimmt sind, die Defizite an Fettsäuren, die für die Emährung von frühgeborenen oder unteremährten Kindern von wesentlicher Bedeutung sind, zu kompensieren.

## Claims

1. Food compositions intended to be directly incorporated in the feeding of very young children **characterized in that** they contain as main constituent phospholipids extracted from mammalian brain, with the exception of those of human, in combination or admixture with a food carrier suitable for the feeding of very young children.

2. Food compositions according to claim 1, **characterized in that** the carrier is a digestible, low-calorie or a higher calorie material.

3. Food compositions according to claim 1 or claim 2, wherein the food carrier is selected among the nutritive element selected from the group consisting of lactose, casein, powdered milk and predigested flours.

4. Food compositions according to claim 1 wherein the food carrier is a sweetened or unsweetened aqueous vehicle.

5. Food compositions according to claim 1 wherein the brain phospholipids are formulated in the form of emulsion.

6. Food compositions according to claim 1, wherein the brain phospholipids are formulated in the form of liposome, the size of which ranges from 100 to 500 µm.

7. Food compositions according to one of the claims 1 to 6 **characterized in that** they are added with other growth factors.

8. Food compositions according to claim 7 wherein the growth factor is a vitamin or a mixture of vitamins.

9. Food compositions according to claim 7 wherein the growth factor is a mineral element selected from the group consisting of calcium salts, magnesium salts, zinc salts and iron salts.

**10.** Food compositions according to one of the claims 1 to 9 wherein the content in brain phospholipids ranges from 1 to 20%.

**11.** A method for producing brain phospholipids according to claim 1 which consists in removing through mechanical means in freshly slaughtered animals the brains, in freezing them immediately at -20°C, in letting the temperature of the organs to rise between -5°C and 0°C before mincing them in an industrial mincing machine then in grinding machines, in order to form a fluid paste the content in water of which is of about 80%, eliminating water from the tissues by transferring the fluid paste at the top of an atomising chamber and rapidly evaporating water in a stream of air super heated at 190-195°C, separating the lipid faction from the resulting powder by extracting it with a mixture of aliphatic $C_6$ -hydrocarbons, at room temperature, filtering the obtained mixture and concentrating to dryness the separated liquid phase under reduced pressure to obtain a crude paste which is poured into acetone, separating the formed precipitate by filtering under nitrogen atmosphere and drying under vacuum the powdered material thus collected.

**12.** Use of brain phospholipids obtained according to the method of claim 11 for achieving food compositions intended for compensating deficiencies in essential unsaturated fatty acids in the feeding of premature or maltrutritionned children.